# EUROPEAN PATENT SPECIFICATION

(11) **EP 1 611 879 B1**
(45) Date of publication and mention of the grant of the patent: **12.08.2009**
(21) Application number: 04291684.1
(22) Date of filing: 02.07.2004
(51) Int. Cl.: A61K 9/107

(54) **Use of emulsions for intra- and periocular injection**
Verwendung von Emulsionen zur intra- und periocularen Injection
Utilisation des émulsions pour injection intra- et périoculaire.

(43) Date of publication of application: 04.01.2006
(73) Proprietor: Novagali Pharma S.A., 91000 Evry (FR); CENTRE NATIONAL DE LA RECHERCHE SCIENTIFIQUE (CNRS), 75016 Paris (FR); INSTITUT NATIONAL DE LA SANTE ET DE LA RECHERCHE MEDICALE (INSERM), 75013 Paris (FR); YISSUM RESEARCH DEVELOPMENT COMPANY OF THE HEBREW UNIVERSITY OF JERUSALEM, 91390 Jerusalem (IL)
(72) Inventor: Rabinovich-Guilatt, Laura, 75015 Paris (FR); De Kozak, Yvonne, 75013 Paris (FR); Dubernet, Catherine, 91360 Epinay-Sur-Orge (FR); Lambert, Gregory, 91370 Verrieres le Buisson (FR); Benita, Simon, 90805 Mevasseret Sion (IL); Couvreur, Patrick, 91140 Bures sur Yvette (FR); Behar-Cohen, Francine, 75016 Paris (FR)
(74) Representative: de Mareüil-Villette, Caroline

(56) References cited:
- EP-A- 0 521 799
- EP-A- 1 020 194
- WO-A-02/09667
- WO-A-93/18852
- WO-A-94/05298
- WO-A-03/053405
- US-A- 5 632 984
- KLANG S H ET AL: "PHYSICOCHEMICAL CHARACTERIAZATION AND ACUTE TOXICITY EVALUATION OF A POSITIVELY-CHARGED SUBMICRON EMULSION VEHICLE" JOURNAL OF PHARMACY AND PHARMACOLOGY, LONDON, GB, vol. 46, no. 12, December 1994 (1994-12), pages 986-993, XP008005426 ISSN: 0022-3573
- KLANG S ET AL: "INFLUENCE OF EMULSION DROPLET SURFACE CHARGE ON INDOMETHACIN OCULAR TISSUE DISTRIBUTION" PHARMACEUTICAL DEVELOPMENT AND TECHNOLOGY, NEW YORK, NY, US, vol. 5, no. 4, 2000, pages 521-532, XP008005503 ISSN: 1083-7450
- ABDULRAZIK M ET AL: "OCULAR DELIVERY OF CYCLOSPORIN A II. EFFECT OF SUBMICRON EMULSION'S SURFACE CHARGE ON OCULAR DISTRIBUTION OF TOPICAL CYCLOSPORIN A" STP PHARMA SCIENCES, PARIS, FR, vol. 11, no. 6, 2001, pages 427-432, XP008033036 ISSN: 1157-1489
- JAFFE G J ET AL: "FLUOCINOLONE ACETONIDE SUSTAINED DRUG DELIVERY DEVICE TO TREAT SEVERE UVEITIS" OPHTHALMOLOGY, J. B. LIPPINCOTT CO., PHILADELPHIA, PA, US, vol. 107, no. 11, November 2000 (2000-11), pages 2024-2033, XP008001875 ISSN: 0161-6420

## Description

The invention generally relates to the use of a composition for intraocular and periocular injections to treat an eye disease.

Delivering therapeutic or diagnostic agents to the posterior segment of the eye, especially to the retina (i.e. the macula) is a challenge.

Providing effective amounts of an agent to, for example, the retina via topical instillation is generally not efficient. Topical delivery of drugs often results in limited ocular absorption due to the complex hydrophobic/hydrophilic properties of the cornea and sclera. The composition tends to be quickly removed from the eye by tears and other natural clearing processes. Topical agents are mechanically removed by the blink mechanism such that only approximately 1-5% of the active compounds contained in single drop are absorbed. This amount is mainly distributed into the anterior segment and then eliminated by the physiological aqueous humour pathways. The resulting amount of active compound reaching the posterior segment most often attains sub-therapeutic levels (Koevary, S.B., Pharmacokinetics of topical ocular drug delivery: potential uses for the treatment of diseases of the posterior segment and beyond. Curr Drug Metab, 2003. 4(3): p. 213-22).

Conversely, ocular absorption of systemically administered pharmacologic agents is limited by the blood ocular barrier, namely the tight junctions of the retinal pigment epithelium and vascular endothelial cells. The barrier limits the size and amount of agents that can reach the choroids and retina. High systemic doses can penetrate this blood ocular barrier in relatively small amounts, but expose the patient to the risk of systemic toxicity. Therefore the dosage is limited so as not to provide a toxic dose of the agent to other parts of the body. This is especially a concern in treating chronic disorders where a long term dosing regimen is typically required.

The simplest way to reach the posterior segment eye tissues is the direct intraocular injection. For the posterior segment of the eye, an intravitreal injection has been used to deliver drugs into the vitreous body. US patent n° 5 632 984 to Wong et al. relates to the treatment of macular degeneration with various drugs by intraocular injection. The drugs are preferably injected as microcapsules. US 5 770 589 to Billson et al. relates to treating macular degeneration by intravitreally injecting an anti-inflammatory into the vitreous humour. While drug is delivered to the posterior segment, it is not specifically administered to a target area such as the macula, but rather is supplied to the entire posterior segment. Additionally, the procedure has a low risk of infection and retinal detachment. Intraocular drug therapy is considered for many disorders, such as retinal detachment, proliferative vitreoretinopathy (PVR), viral retinitis and uveitis, macular edema (of any origin), proliferation of neo vessels from the retina and/or from the choroid, intraocular inflammation and retinal degenerations (retinal dystrophies).

The short half-life of some of the therapeutic agents compels the intravitreal injections to be repeated daily or weekly to maintain therapeutic levels. Several methods of sustained drug-release are currently being investigated to aid in this therapy, with two main products on the market (Vitrasert and Vitravene). These new delivery systems include drug entrapment in liposomes, microspheres, and polymeric materials which control the release of the drug and sustain therapeutic levels over an extended period of time (Slatker, JS et al. (2003). Anecortave acetate as monotherapy for treatment of subfoveal neovascularization in age-related macular degeneration: twelve-month clinical outcomes. Ophthalmology 110 (12): 2372-2383. Tan, DT et al. (2001). Randomized clinical trial of Surodex steroid drug delivery system for cataract surgery anterior versus posterior placement of two Surodex in the eye. Ophthalmology 108 (12): 2172-2181. Jaffe, G et al. (2000). Fluocinolone acetonide sustained drug delivery device to treat severe uveitis. Ophthalmology 107 (11): 2024-2033. Diaz-Llopis, M et al. (1992). Liposomally-entrapped gan ciclovir for the treatment of cytomegalovirus retinitis in AIDS patients. Experimental toxicity and pharmacokinetics, and clinical trial. Peyman GA, et al. (1992). Clearance of microsphere-entrapped 5-fluorouracil and cytosine arabinoside from the vitreus of primates). US 5,378,475 describes a device which has been developed for insertion in the vitreous region of the eye, as published in T.J. Smith et al., Sustained-Release Ganciclovir, Arch. Ophthalmol, 110, 255-258 (1992) and G. E. Sanborn, et al., Sustained-Release Ganciclovir Therapy for Treatment of Cytomegalovirus Retinitis. Use of an Intravitreal Device, Arch. Ophthalmol, 110, 188-195 (1992). US 5,098,443 describes certain specific implants that are inserted through incisions made in the eye wall or ,sutured around the globe of the eye. These rings may be formed from biodegradable polymers containing microparticles of drug. Initial results indicate that these technologies do retard drug release after injection, but they introduce other problems, such as intravitreal toxicity of the drug carriers, interference with vision and difficulties in the large-scale manufacture of sterile preparations, in addition to their high cost of production.

Drug delivery using liposomes and microspheres may be useful in treating posterior segment disease; however, there are also inherent problems encountered with these methods, including manufacturing complexity, sterilisation and production cost. These formulations also spread diffusely within the vitreous cavity and causes cloudiness, interfering with the patient's visual acuity and the ability of the ophthalmologist to examine the fundus until complete resorption of the formulation has occurred 14-21 days after administration. These problems have led investigators to examine other modes of delivery systems.

Despite all the mentioned above, there is still no pharmaceutical formulation (excipient) specifically adapted for intraocular administration. While for hydrophilic molecules this concerns may be easily resolved by administering a sterile isotonic aqueous solution, when the therapeutic agent is hydrophobic the physician is obliged to inject unsuitable products (Nishimura, A et al. 2003. Isolating triamcinolone acetonide particles for intravitreal use with a porous membrane filter. Retina 23, 777-779) .

The present invention provides the use of a composition comprising an emulsion and optionally at least a pharmaceutical active ingredient for the manufacture of a medicament in a form adapted for intraocular and periocular administration according to claim 1.

According to the instant invention, intraocular administration is intravitreal administration and periocular administration comprises peribulbar, laterobulbar, subconjonctival, sub-tenon and retrobulbar administration.

According to the instant invention, the emulsion is preferably an oil/water type emulsion.

Also disclosed is a method for treating eye diseases by injecting intraocularely or periocularely a composition comprising an emulsion and optionally at least a pharmaceutical active ingredient.

The emulsion is preferably selected from the group comprising anionic and cationic emulsions. Examples of cationic emulsions are the emulsions disclosed in WO 93/18852, i.e. oil/water type emulsion which comprises colloid particles having an oily core surrounded by an interfacial film, the film comprising surface active agents, lipids or both, said emulsions being characterised in that at least part of the surface active agents or lipids in the interfacial film have positively charged polar groups and further in that the colloid particles have a positive zeta potential. The interfacial film may also comprise non-ionic surfactants or lipids.

Examples of anionic emulsions are the emulsions described in Klang, S et al. 2 000. Influence of emulsion droplet surface charge on indomethacin ocular tissue distribution. Pharm Dev Technol 5(4): p.521-32 and Abdulrazik, M, et al. 2001. Ocular delivery of cyclosporin A. II. Effect of submicron emulsion's surface charge on ocular distribution of topical cyclosporin A. STP Pharma Sciences 11(6): p.427-432., i.e. oil/water type emulsion which comprises colloid particles having an oily core surrounded by an interfacial film, the film comprising surface active agents, lipids or both, said emulsions being characterised in that at least part of the surface active agents or lipids in the interfacial film have negatively charged polar groups and further in that the colloid particles have a negative zeta potential. The interfacial film may also comprise non-ionic surfactants or lipids.

In order to have a positive zeta potential the total amount of charges of the cationic agents should be in excess to the total amount of charges of the anionic agents. In order to have a negative zeta potential the total amount of charges of the anionic agents should be in excess to the total amount of charges of the cationic agents.

Examples of cationic lipids are C₁₀-C₂₄-alkylamines and C₁₂-C₂₄-alkanolamines, C₁₂-C₁₈-alkylamines and C₁₂-C₁₈-alkanolamines being preferred. Specific examples of cationic lipids are stearylamine, oleylamine and cholesteryl betainate and various cationic cholesterol esters and derivatives.

Examples of anionic lipids are phospholipids. The examples of phospholipids, which may be used in the emulsions of the invention, are lecithins; Epikuron 120.™ (Lucas Meyer, Germany) which is a mixture of about 70% phosphatidylcholine and 12% phosphatidylethanolamine and about 15% other phospholipids; Ovothin 160.™ or Ovothin 200.™ (Lucas Meyer, phosphatidylcholine, 18% phosphatidylethanolamine and 12% other phospholipids; a purified phospholipid mixture, e.g. such which is obtained from egg yolk; Lipoid E-80.™ (Lipoid AG, Ludwigshafen, Germany) which is a phospholipid mixture comprising about 80% phosphatidylcholine, 8% phosphatidylethanolamine, 3.6% non-polar lipids and about 2% sphingomyeline.

Examples of anionic surfactants, which may be included, are sodium lauryl sulphate and alkylpolyoxyethelene sulphate and sulfonate.

Examples of non-ionic surfactants, which may be included in the emulsion of the invention, poloxamers such as Pluronic F-68LF.™, Pluronic L-62LF.™ and Pluronic L62D.™ (BASF Wyandotte Corp., Parsippany, N.J., USA), polysorbate such as polysorbate 80, polyoxyethylene fatty acid esters such as EMULPHOR.™ (GAF Corp., Wayne, N.J., USA).

The oily phase of the emulsion may comprise one or more members selected from the group consisting of vegetable oil (i.e. soybean oil, olive oil, sesame oil, cotton seed oil, castor oil, sweet almond oil), mineral oil (i.e. petrolatum and liquid paraffin), medium chain triglycerides (MCT) oil (i.e. a triglyceride oil in which the carbohydrate chain has about 8-12 carbon atoms), oily fatty acid, isopropyl myristate, oily fatty alcohols, esters of sorbitol and fatty acids, oily sucrose esters, and in general any oily substance which is physiologically tolerated.

The major component of the oily phase will generally be either vegetable oil and/or MCT. Fatty acids or fatty alcohols may be included in cases where the hydrophobic substance to be carried by the emulsion is not sufficiently soluble in the oily phase.

Examples of MCT oil, which may be used in emulsions of the present invention, are TCM.™ (Société des Oléagineux, France), Miglyol 812.™ (Dynamit Novel, Sweden).

Examples of oily fatty acids, which may be used in emulsions of the invention, are oleic acid, linoleic acid, linolenic acid, palmitic acid, arachidonic acid, lauric acid and others. Examples of fatty alcohols, which may be used, are oleyl alcohol, cetyl alcohol and others. Examples of esters of sorbitol and fatty acids are sorbitan monooleate and sorbiton mono-palmitate. Examples of oily sucrose esters are sucrose mono-, di-or tri-palmitate.

As known, the emulsion may also comprise various additives such as osmotic pressure regulators, e.g. sucrose, glycerine or mannitol; anti-oxidants, e.g. alpha-tocopherol, ascorbic acid or ascorbyl palmitate; or preservatives, e.g. methyl-, ethyl-, and butyl paraben. In some applications, other additives may further be included in the substance and, for example, some suitable additives may include dextrose, carriers, stabilizing agents, wetting agents, viscosity enhancers, hydrogels or other similar materials.

The preferred ranges of ingredients in the emulsion according to the invention are (expressed in % w/w): oily carrier: 0.5-20%, 0.5-10% being particularly preferred; cationic or anionic surfactants or lipids: 0.01-2%, 0.02-0.4% being particularly preferred and optionally non-ionic surfactant: 0.05-3%, its preferred range being 0.1-2%. Where the emulsion comprises phospholipids: 0.05-3%, 0.1-2% being particularly preferred. These preferred ranges are to be understood as standing each by itself and not cumulative.

A preferred pH in the aqueous phase of the emulsion of the invention is 4.0-8.5, 6.0-8.0 being particularly preferred.

It is generally preferred that the particles in the emulsion will have a diameter below about 300 nanometers, a diameter less than 200 nanometers being particularly preferred.

In the oil/water emulsion, the water-insoluble drug is solubilised in the internal oil phase, thereby remaining in the preferred solution state. In addition, the blurred vision caused by oils is minimised by the water in the external phase. Furthermore, the concentration of the drug in the oil phase can be adjusted to maximise thermodynamic activity, thus enhancing drug penetration to deeper tissues.

A wide variety of ocular conditions such as intraocular inflammation, infection, cancerous growth, tumors, neo vessel growth originating from the retina and/ or from the choroids, retinal edema, macular edema, diabetic retinopathy, retinopathy of prematurity, degenerative diseases of the retina (macular degeneration, retinal dystrophies), retinal diseases associated with glial proliferation, more specifically, ocular conditions such as glaucoma, proliferative vitreoretinopathy, diabetic retinopathy, age-related macular degeneration, uveitis, cytomegalovirus retinitis, herpes simplex viral retinal dystrophies, age related macular degeneration may be prevented or treated using the cationic or anionic emulsions according to the present invention.

Some substances suitable for delivery to the eye may include, for example, anaesthetics, analgesics, cell transport/mobility impending agents such as colchicines, vincristine, cytochalasin B and related compounds; carbonic anhydrase inhibitors such as acetazolamide, methazolamide, dichlorphenamide, diamox and neuroprotectants such as nimodipine and related compounds; antibiotics such as tetracycline, chlortetracycline, bacitracin, neomycin, polymyxin, gramicidin, cephalexin, oxytetracycline, chloramphenicol, rifampicin, ciprofloxacin, aminosides, gentamycin, erythromycin and penicillin, quinolone, ceftazidime, vancomycine imipeneme; antifungals such as amphotericin B, fluconazole, ketoconazole and miconazole; antibacterials such as sulfonamides, sulfadiazine, sulfacetamide, sulfamethizole and sulfisoxazole, nitrofurazone and sodium propionate; antivirals, such as idoxuridine, trifluorothymidine, trifluorouridine, acyclovir, ganciclovir, cidofovir, interferon, DDI, AZT, foscamet, vidarabine, irbavirin, protease inhibitors and anti-cytomegalovirus agents; antiallergenics such as sodium cromoglycate, antazoline, methapyriline, chlorpheniramine, cetirizine, pyrilamine and prophenpyridamine; synthetic gluocorticoids and mineralocorticoids and more generally hormones forms derivating from the cholesterol metabolism (DHEA, progesterone, estrogens); non-steroidal anti-inflammatories such as salicylate, indomethacin, ibuprofen, diclofenac, flurbiprofen, piroxicam and COX2 inhibitors; antineoplastics such as carmustine, cisplatin, fluorouracil; adriamycin, asparaginase, azacitidine, azathioprine, bleomycin, busulfan, carboplatin, carmustine, chlorambucil, cyclophosphamide, cyclosporine, cytarabine, dacarbazine, dactinomycin, daunorubicin, doxorubicin, estramustine, etoposide, etretinate, filgrastin, floxuridine, fludarabine, fluorouracil, florxymesterone, flutamide, goserelin, hydroxyurea, ifosfamide, leuprolide, levamisole, limustine, nitrogen mustard, melphalan, mercaptopurine, methotrexate, mitomycin, mitotane, pentostatin, pipobroman, plicamycin, procarbazine, sargramostin, streptozocin, tamoxifen, taxol ,teniposide, thioguanine, uracil mustard, vinblastine, vincristine and vindesine; immunological drugs such as vaccines and immune stimulants; insulin, calcitonin, parathyroid hormone and peptide and vasopressin hypothalamus releasing factor; beta adrenergic blockers such as timolol, levobunolol and betaxolol; cytokines, interleukines and growth factors epidermal growth factor, fibroblast growth factor, platelet derived growth factor, transforming growth factor beta, ciliary neurotrophic growth factor, glial derived neurotrophic factor, NGF, EPO, PLGF, brain nerve growth factor (BNGF), vascular endothelial growth factor (VEGF) and monoclonal antibodies directed against such growth factors; anti-inflammatories such as hydrocortisone, dexamethasone, fluocinolone, prednisone, prednisolone, methylprednisolone, fluorometholone, betamethasone and triamcinolone; decongestants such as phenylephrine, naphazoline and tetrahydrazoline; miotics and anti-cholinesterases such as pilocarpine, carbachol, di-isopropyl fluorophosphate, phospholine iodine and demecarium bromide; mydriatics such as atropine sulphate, cyclopentolate, homatropine, scopolamine, tropicamide, eucatropine; sympathomimetics such as epinephrine and vasoconstrictors and vasodilators. Anticlotting agents such as heparin, antifibrinogen, fibrinolysin, anticlotting activase, antidiabetic agents include acetohexamide, chlorpropamide, glipizide, glyburide, tolazamide, tolbutamide, insulin and aldose reductase inhibitors, hormones, peptides, nucleic acids, saccharides, lipids, glycolipids, glycoproteins and other macromolecules include endocrine hormones such as pituitary, insulin, insulin-related growth factor, thyroid, growth hormones; heat shock proteins; immunological response modifiers such as muramyl dipeptide, cyclosporins, interferons (including alpha-, beta- and gamma- interferons), interleukin-2, cytokines, FK506 (an epoxy-pyrido-oxaazcyclotricosine-tetrone, also known as Tacrolimus), tumor necrosis factor, pentostatin, thymopentin, transforming factor beta. sub. 2, erythropoetin; antineogenesis proteins (e.g. anti VEGF, Interfurons).

Antibodies (monoclonal or polyclonal) or antibodies fragments, oligoaptamers, aptamers and gene fragments (oligonucleotides, plasmids, ribozymes, small interference RNA (SiRNA), nucleic acid fragments, peptides).

Immunomodulators such as endoxan, thalidomide, tamoxifene. Antithrombolytic and vasodilator agents such as rtPA, urokinase, plasmin, Nitric oxide donors.

In addition, nucleic acids can also be delivered wherein the nucleic acid may be expressed to produce a protein that may have a variety of pharmacological, physiological or immunological activities.

The invention is further illustrated by the examples and the figures.
**Figure 1** describes the ocular inflammation evolution following the intravitreal administration.
**Figure 2** illustrates mean inflammation clinical score (A) and retinal injure (B) of the untreated and treated groups.

### Example 1: Preparation of compositions

**Composition 1: Cationic emulsion with cyclosporine A**

| | |
|---|---|
| Cyclosporine A (active) | 0.2% |
| Oleylamine (cationic lipid) | 0.1% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

Size: 150 nm
Zeta: +58mV
Osmolarity: 290 mosm
pH adjusted at 8.0 with HCL 0.1N
pH after autoclave = 7.3
Oily phase components and cyclosporine A are successively weighed in the same beaker and then magnetically stirred under a slight heating (40°C) until a yellow, limpid and slightly viscous phase is obtained. Aqueous phase components are successively weighed in the same beaker and then magnetically stirred under a slight heating (40°C) until a transparent, limpid and fluid phase is obtained. Both phases are heated to 65°C. The coarse emulsion is formed by rapid addition of the aqueous phase in the oily phase and is then rapidly heated to 75°C. The aqueous phase and coarse emulsion beakers are protected by a film to avoid any water evaporation. The emulsion is white and slightly transparent. The emulsion droplet's size is then decreased by a 5 minutes high shear mixing with POLYTRON PT 6100. The emulsion becomes milky. The emulsion temperature is cooled down to 20°C using an ice bath. The final emulsion is obtained by homogenization in a microfluidizer (C5, Avestin) using continuous cycles for 5 min at a pressure of 10 000 psi. The emulsion is milky, very fluid and does not adhere on the glass. The emulsion temperature is decreased to 25°C. Its pH is measured and then adjusted to 8.00 using a 0.1M HCl solution. Emulsion is conditioned in tinted glass vials with nitrogen bubbling and then sterilized in an autoclave 20 minutes at 121°C.

**Composition 2: Cationic emulsion without pharmaceutical active ingredient.**

| | |
|---|---|
| Oleylamine (cationic lipid) | 0.1% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

**Composition 3 (Comparative): Anionic emulsion with cyclosporine A**

| | |
|---|---|
| Cyclosporine A (active) | 0.2% |
| Deoxycholic acid | 0.1% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

**Composition 4 (Comparative): Anionic emulsion with cyclosporine A**

| | |
|---|---|
| Cyclosporine A (active) | 0.2% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

**Composition 5 (Comparative): Anionic emulsion without pharmaceutical active ingredient**

| | |
|---|---|
| Oleylamine (cationic lipid) | 0.1% |
| Deoxycholic acid | 0.1% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

**Composition 6: Cationic emulsion with thalidomide**

| | |
|---|---|
| Thalidomide (active) | 0.1% |
| Oleylamine (cationic lipid) | 0.1% |
| paraffin (oil) | 2% |
| alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

**Composition 7: Cationic emulsion with medroxyprogesterone**

| | |
|---|---|
| Medroxyprogesterone acetate (active) | 0.05% |
| Oleylamine (cationic lipid) | 0.2% |
| Soybean oil (oil) | 5% |
| Ascorbyl palmitate (antioxidant) | 0.05% |
| Lipoid E80 (surfactant) | 0.5% |
| Lutrol F68 (surfactant) | 0.8% |
| Mannitol (tonicity agent) | 1% |
| Water | QSP 100% |

**Composition 8: Cationic emulsion with triamcinolone acetonide**

| | |
|---|---|
| Triamcinolone acetonide (active) | 0.05% |
| Oleylamine (cationic lipid) | 0.1% |
| Castor oil (oil) | 8% |
| Alpha tocopherol (antioxidant) | 0.05% |
| Lipoid E80 (surfactant) | 1% |
| Lutrol F68 (surfactant) | 0.8% |
| Mannitol (tonicity agent) | 1% |
| Water | QSP 100% |

**Composition 9: Cationic emulsion with dexamethasone**

| | |
|---|---|
| Dexamethasone (active) | 0.16% |
| Oleylamine (cationic lipid) | 0.1% |
| MCT (oil) | 2% |
| Alpha tocopherol (antioxidant) | 0.01% |
| Lipoid E80 (surfactant) | 0.32% |
| Lutrol F68 (surfactant) | 0.5% |
| Glycerine (tonicity agent) | 2.25% |
| Water | QSP 100% |

All compositions 2 to 9 were prepared like composition 1.

### Example 2: Safety of the cationic emulsions administered intravitrealy (IVT) to healthy animals.

### 2.1. Method.

10 µL of either saline, empty cationic emulsion (composition 2) or cationic emulsion containing 0.2% cyclosporine A (CsA) (composition 1) were administered IVT to anaesthetized male Lewis rats (8-11 weeks old) (n=2/group).
The potential proinflammatory effect of empty cationic emulsions or CsA-cationic emulsions was assessed at 1 and 7 days post-injection by means of a slit lamp according to the scale disclosed by Thillaye-Goldenberg B. et al., Delayed onset and decreased severity of experimental autoimmune uvoeretinitis in mice lacking nitric oxide synthase type 2. in J. Neuroimmunol., 2000, vol. 110, 31-44. The score is 0 to 4 according to the following table:

| *Clinical examination* | *Score* |
|---|---|
| Full iris dilatation, no cell in the aqueous humeur or vitreous | 0 |
| Partial dilation (mild synechiae) | 1 |
| Moderate dilatation (moderate synechiae) | 2 |
| Severer synechiae + hypopion | 3 |
| As previous + fibrine plugging | 4 |

### 2.2. Results.

### 2.2.1. Ocular inflammation.

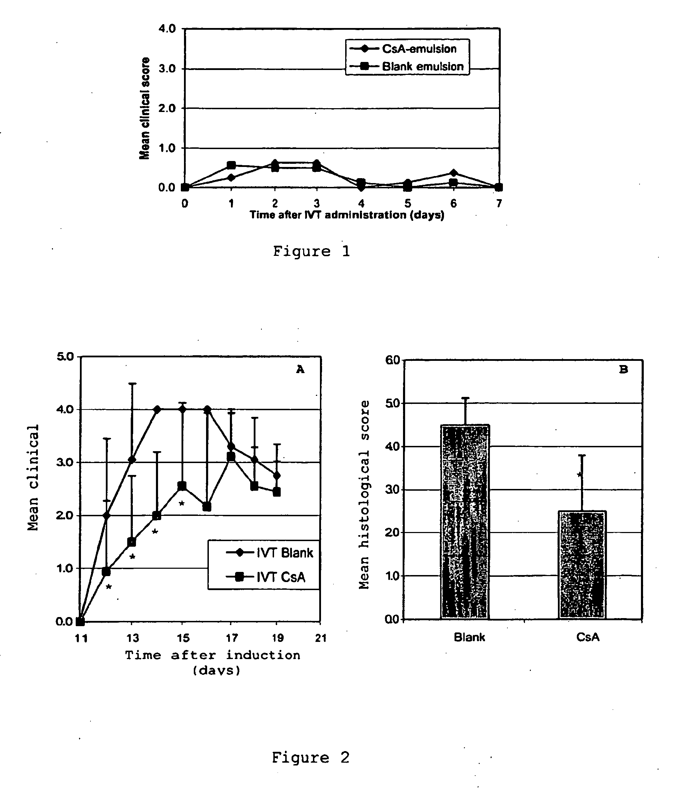

The results are shown in figure 1.
Intravitreal administration of blank emulsion or CsA emulsion do not lead to ocular inflammation.

### Example 3: Safety of the cationic emulsions administered subconjunctivally to animals.

### 3.1. Method.

Twenty µL of empty cationic emulsion (composition 2) or cationic emulsion containing 0.2% cyclosporine A (CsA) (composition 1) were administered subconjunctivally to anaesthetized male Lewis rats (n=2/group).

### 3.2. Results.

Subconjunctival administration of blank emulsion or CsA emulsion does not lead to any appreciable proinflammatory effect.

### Example 4: Efficacy of the CsA-containing emulsion administered IVT in an animal model of posterior uveitis.

### 4.1. Model.

Experimental Autoimmune Uveitis (EAU) is an ocular inflammatory disease induced in rodents which serves as a model for the human posterior uveitis (Caspi, RR. et al., 1988. A new model of autoimmune disease: experimental autoimmune uveoretinitis induced in mice with two difference retinal antigens. J. Immunol. 140: 1490-1495). The onset and duration of the experimental disease are dependent on the Ag immunizing dose, animal species and type of adjuvant. It is a CD4⁺T-cell driven disease, leading in one month to destruction of photoreceptor cells and blindness. The ocular inflammation starts at day 12-13 after the systemic immunization with purified retinal autoantigens in adjuvants by an inflammatory cell infiltration, thereafter at 30 days agter immunisation (with Th2-anti-inflammatory: IL-4, IL-10, TGF-β-cytokines playing a role with an increase in Th1 (proinflammatory: IFN-γ, TNF-α, IL-2) cytokines, reaches a peak, and decreases in the resolution of the disease).
This immune reaction results in the destruction of the photoreceptor cell, target of the immune response. Although both humoral and cellular immune reactions are stimulated in patients and in animal models, the cell-mediated immunity plays the main role as already described, and therefore there is a rational for CsA administration. Indeed, several studies have demonstrated the therapeutical efficacy of systemic CsA in human uveitis, but the side effects associated to a chronic systemic administration of CsA prevent its extensive use.

### 4.2. Methods.

### 4.2.1. Administration.

10 µL of either empty cationic emulsion (composition 2) or cationic emulsion containing 0.2% CsA (composition 1) were administered IVT to anaesthetised male Lewis rats (n=5/group).

### 4.2.2. Ocular inflammation.

The ocular inflammation was assessed during the experimental period as previously described for toxicity experiments in example 1. Significance was evaluated for each time point by the non-parametric Mann-Whitney U test.
Probability values <0.05 were considered significant.

### 4.2.3. Histopathology.

The retinal damage was evaluated at the time of sacrifice after hematein/eosin/safran in staining under optical microscopy and according to the following score (Ramanathan S. et al., Recombinant IL-4 aggravates experimental autoimmune uveoretinitis in rats in J. Immunol., 1996, vol. 157, 2209-2215).

| Score | Observation |
|---|---|
| 0 | Normal retina |
| 1 | Partially damaged extracellular segments |
| 2 | Fully damaged extracellular segments |
| 3 | Partially damaged photoreceptors nuclei |
| 4 | Fully damaged photoreceptors nuclei |
| 5 | Partially damaged bipolar cells nuclei |
| 6 | Fully damaged bipolar cells nuclei |
| 7 | Damage to the ganglion cells |

Significance was evaluated by the non-parametric Mann-Whitney U test. Probability values < 0.05 were considered significant.

### 4.3. Results.

### 4.4.1. Clinical inflammation.

The administration of cyclosporine in the form of a 0.2% emulsion reduced significantly the clinical score of the inflamed eyes during the first days post-injection, compared to the blank emulsion (Figure 2A). For example, at D14 the treated eyes showed a mean value of 1.8 ± 1.3 while all the untreated ones exhibited scores of 4.0. The blank emulsion by itself had no effect compared to saline.

### 4.4.2. Retinal injury.

The retinal damage as evaluated from the examination of histological slides (Figure 2B) suggests that the blank emulsion ameliorates EAU, as from 2.8 ± 1.7 (saline group) the score was lowered to 1.0 ± 0.4 (blank emulsion). Incorporation of CsA to the emulsion further reduced the damage to 0.1 ± 0.3.

## Claims

1. Use of a composition comprising:
- a cationic emulsion, which is an oil/water type emulsion which comprises colloid particles having an oily core surrounded by an interfacial film, the film comprising surface active agents, lipids or both, said emulsions being **characterised in that** at least part of the surface active agents or lipids in the interfacial film have positively charged polar groups and further **in that** the colloid particles have a positive zeta potential, and
- optionally at least a pharmaceutical active ingredient
for the manufacture of a medicament in a form adapted for intra- and periocular administration for treating intraocular conditions selected from the group of intraocular inflammation, infection, cancerous growth, tumors, neo vessel growth originating from the retina and/ or from the choroids, retinal edema, macular edema, diabetic retinopathy, retinopathy of prematurity, degenerative diseases of the retina (macular degeneration, retinal dystrophies), retinal diseases associated with glial proliferation, more specifically, ocular conditions such as glaucoma, proliferative vitreoretinopathy, diabetic retinopathy, age-related macular degeneration, uveitis, cytomegalovirus retinitis, herpes simplex viral retinal dystrophies, by intravitreal, peribulbar, laterobulbar, subconjonctival, subtimor and retrobulbar administration.

2. Use according to claim 1, wherein the cationic emulsion comprises (expressed in % w/w): 0.5-20% oily carrier, preferably 0.5-10%; 0.01-2% cationic surfactants or lipids, preferably 0.02-0.4% and optionally a non ionic surfactant in a range of 0.05-3%, preferably in a range of 0.1-2%.

3. Use according to claim 2, wherein the emulsion comprises further 0.05-3% of phospholipids, preferably 0.1-2%.

4. Use according to anyone of claims 1 to 3, wherein the pH of the emulsion is comprised between 4.0 and 8.5, preferably between 6.0 and 8.0.

5. Use according to anyone of claims 1 to 4, wherein the emulsion further comprises additive selected from the group of osmotic pressure regulators, antioxidants, preservatives, dextrose, carriers, stabilizing agents, wetting agents, viscosity enhancers, hydrogels.

6. Use according to anyone of claims 1 to 5, wherein the active ingredient is selected from the group of anaesthetics, analgesics, cell transport/ mobility impending agents such as colchicines, vincristine, cytochalasin B and related compounds; carbonic anhydrase inhibitors chosen from the group comprising acetazolamide, methazolamide, dichlorphenamide, diamox and neuroprotectants chosen from the group comprising nimodipine and related compounds; antibiotics chosen from the group comprising tetracycline, chlortetracycline, bacitracin, neomycin, polymyxin, gramicidin, cephalexin, oxytetracycline, chloramphenicol, rifampicin, ciprofloxacin, aminosides, gentamycin, erythromycin and penicillin, quinolone, ceftazidime, vancomycine imipeneme; antifungals chosen from the group comprising amphotericin B, fluconazole, ketoconazole and miconazole; antibacterials chosen from the group comprising sulfonamides, sulfadiazine, sulfacetamide, sulfamethizole and sulfisoxazole, nitrofurazone and sodium propionate; antivirals, chosen from the group comprising idoxuridine, trifluorothymidine, trifluorouridine, acyclovir, ganciclovir, cidofovir, interferon, DDI, AZT, foscamet, vidarabine, irbavirin, protease inhibitors and anti-cytomegalovirus agents; antiallergenics chosen from the group comprising sodium cromoglycate, antazoline, methapyriline, chlorpheniramine, cetirizine, pyrilamine and prophenpyridamine; synthetic gluocorticoids and mineralocorticoids and more generally hormones forms derivating from the cholesterol metabolism (DHEA, progesterone, estrogens); non-steroidal antiinflammatories chosen from the group comprising salicylate, indomethacin, ibuprofen, diclofenac, flurbiprofen, piroxicam and COX2 inhibitors; antineoplastics chosen from the group comprising carmustine, cisplatin, fluorouracil; adriamycin, asparaginase, azacitidine, azathioprine, bleomycin, busulfan, carboplatin, carmustine, chlorambucil, cyclophosphamide, cyclosporine, cytarabine, dacarbazine, dactinomycin, daunorubicin, doxorubicin, estramustine, etoposide, etretinate, filgrastin, floxuridine, fludarabine, fluorouracil, florxymesterone, flutamide, goserelin, hydroxyurea, ifosfamide, leuprolide, levamisole, limustine, nitrogen mustard, melphalan, mercaptopurine, methotrexate, mitomycin, mitotane, pentostatin, pipobroman, plicamycin, procarbazine, sargramostin, streptozocin, tamoxifen, taxol , teniposide, thioguanine, uracil mustard, vinblastine, vincristine and vindesine; immunological drugs chosen from the group comprising vaccines and immune stimulants; insulin, calcitonin, parathyroid hormone and peptide and vasopressin hypothalamus releasing factor; beta adrenergic blockers chosen from the group comprising timolol, levobunolol and betaxolol; cytokines, interleukines and growth factors, epidermal growth factor, fibroblast growth factor, platelet derived growth factor, transforming growth factor beta, ciliary neurotrophic growth factor, glial derived neurotrophic factor, NGF, EPO, PLGF, brain nerve growth factor (BNGF), vascular endothelial growth factor (VEGF) and monoclonal antibodies directed against such growth factors; antiinflammatories chosen from the group comprising hydrocortisone, dexamethasone, fluocinolone, prednisone, prednisolone, methylprednisolone, fluorometholone, betamethasone and triamcinolone; decongestants chosen from the group comprising phenylephrine, naphazoline and tetrahydrazoline; miotics and anti-cholinesterases chosen from the group comprising pilocarpine, carbachol, diisopropyl fluorophosphate, phospholine iodine and demecarium bromide; mydriatics chosen from the group comprising atropine sulphate, cyclopentolate, homatropine, scopolamine, tropicamide, eucatropine; sympathomimetics chosen from the group comprising epinephrine and vasoconstrictors and vasodilators; anticlotting agents chosen from the group comprising heparin, anti fibrinogen, fibrinolysin, anticlotting activase, antidiabetic agents chosen from the group comprising acetohexamide, chlorpropamide, glipizide, glyburide, tolazamide, tolbutamide, insulin and aldose reductase inhibitors, hormones, peptides, nucleic acids, saccharides, lipids, glycolipids, glycoproteins and other macromolecules including endocrine hormones chosen from the group comprising pituitary, insulin, insulin-related growth factor, thyroid, growth hormones; heat shock proteins; immunological response modifiers chosen from the group comprising muramyl dipeptide, cyclosporins, interferons (including alpha-, beta- and gamma-interferons), interleukin-2, cytokines, FK506 (an epoxy-pyrido-oxaazcyclotricosine-tetrone, also known as Tacrolimus), tumor necrosis factor, pentostatin, thymopentin, transforming factor beta.sub.2, erythropoetin; antineogenesis proteins (e.g. anti VEGF, Interferons); antibodies (monoclonal or polyclonal) or antibodies fragments, oligoaptamers, aptamers and gene fragments (oligonucleotides, plasmids, ribozymes, small interference RNA (SiRNA), nucleic acid fragments, peptides); immunomodulators chosen from the group comprising endoxan, thalidomide, tamoxifene; antithrombolytic and vasodilator agents chosen from the group comprising rtPA, urokinase, plasmin, Nitric oxide donors; nucleic acids optionally expressed to produce a protein that may have a variety of pharmacological, physiological or immunological activities.

## Patentansprüche

1. Verwendung einer Zusammensetzung, umfassend:
- eine kationische Emulsion, die eine Emulsion des Öl/Wasser-Typs ist, die Kolloidteilchen umfasst mit einem öligen Kern, der umgeben ist von einem Zwischenfilm, wobei der Film oberflächenaktive Mittel, Lipide oder beides umfasst, wobei die Emulsionen **dadurch gekennzeichnet sind, dass** mindestens ein Teil der oberflächenaktiven Mittel oder Lipide in dem Zwischenfilm positiv geladene polare Gruppen aufweist und weiterhin dadurch, dass die Kolloidteilchen ein positives Zeta-Potential haben, und
- optional wenigstens einen pharmazeutisch aktiven Bestandteil, zur Herstellung eines Medikaments in einer Form, die geeignet ist zur intra- und periokulären Verabreichung zur Behandlung von intraokularen Zuständen, ausgewählt aus der Gruppe von intraokularer / Entzündung, intraokularer Infektion, intraokularem krebsartigem Wachstum, intraokularen Tumoren, intraokularem Neogefäßwachstum, das von der Retina und/oder von den Chorioideas ausgeht, Retinalödem, Makulaödem, diabetischer Retinopathie, Retinopathie von Prämaturität, degenerativen Krankheiten der Retina (Makuladegeneration, Retinadystrophien), Retinakrankheiten, die assoziiert sind mit Glialproliferation, insbesondere Okularzustände, wie etwa Glaukom, proliferative Vitreoretinopathie, diabetische Retinopathie, altersbedingte Makuladegeneration, Uveitis, Zytomegalovirus-Retinitis, virale Herpes simplex-Retinadystrophien, durch intravitreale, peribulbäre, laterobulbäre, subkonjunktivale, subtimore und retrobulbäre Verabreichung.

2. Verwendung nach Anspruch 1, worin die kationische Emulsion umfasst (ausgedrückt in % Gew./Gew.): 0,5-20% öligen Träger, vorzugsweise 0,5-10%; 0,01-2% kationische oberflächenaktive Mittel oder Lipide, vorzugsweise 0,02-0,4%, und optional ein nichtionisches oberflächenaktives Mittel in einem Bereich von 0,05-3%; vorzugsweise in einem Bereich von 0,1-2%.

3. Verwendung nach Anspruch 2, worin die Emulsion weiterhin 0,05-3%, vorzugsweise 0,1-2%, Phospholipide umfasst.

4. Verwendung nach einem der Ansprüche 1 bis 3, worin der pH-Wert der Emulsion zwischen 4,0 und 8,5, vorzugsweise zwischen 6,0 und 8,0, liegt.

5. Verwendung nach einem der Ansprüche 1 bis 4, worin die Emulsion weiterhin Additive umfasst, ausgewählt aus der Gruppe von Regulatoren für osmotischen Druck, Antioxidationsmitteln, Konservierungsmitteln, Dextrose, Träger, Stabilisierungsmittel, Benetzungsmittel, Viskositätsverbesserungsmittel, Hydrogele.

6. Verwendung nach einem der Ansprüche 1 bis 5, worin der aktive Bestandteil ausgewählt wird aus der Gruppe von Anästhetika, Analgetika, Zelltransport/Mobilität-Beeinträchtigungsmittel, wie etwa Colchicin, Vincristin, Cytochalasin B und verwandte Verbindungen; Carboanhydrase-Inhibitoren, ausgewählt aus der Gruppe umfassend Azetazolamid, Methazolamid, Dichlorphenamid, Diamox, und Neuroprotektoren, ausgewählt aus der Gruppe umfassend Nimodipin und verwandte Verbindungen; Antibiotika, ausgewählt aus der Gruppe umfassend Tetracyclin, Chlortetracyclin, Bacitracin, Neomycin, Polymyxin, Gramicidin, Cephalexin, Oxytetracyclin, Chloramphenicol, Rifampicin, Ciprofloxacin, Aminoside, Gentamycin, Erythromycin und Penicillin, Quinolon, Ceftazidim, Vancomycin, Imipenem, Antipilzmittel, ausgewählt aus der Gruppe umfassend Amphotericin B, Fluconazol, Ketoconazol und Miconazol; Antibakterielle Mittel, ausgewählt aus der Gruppe umfassend Sulfonamide, Sulfadiazin, Sulfacetamid, Sulfamethizol und Sulfisoxazol, Nitrofuazon und Natriumpropionat; antivirale Mittel, ausgewählt aus der Gruppe umfassend Idoxuridin, Trifluorthymidin, Trifluoruridin, Acyclovir, Ganciclovir, Cidofovir, Interferon, DDI, AZT, Foscamet, Vidarabin, Irbavirin, Proteaseinhibitoren und Antizytomegalovirus-Mittel; Antiallergiemittel, ausgewählt aus der Gruppe umfassend Natriumcromoglycat, Antazolin, Methapyrilin, Chlorpheniramin, Cetirizin, Pyrilamin und Prophenpyridamin; synthetische Glucocorticoide und Mineralocorticoide und allgemeiner Hormonformen, die vom Cholesterolmetabolismus stammen (DHEA, Progesteron, Östrogene); Nicht-Steroid-Antientzündungsmittel, ausgewählt aus der Gruppe, umfassend Salicylat, Indomethacin, Ibuprofen, Diclofenac, Flurbiprofen, Piroxicam und COX2-Inhibitoren; Antineoplasiemittel, ausgewählt aus der Gruppe umfassend Carmustin, Cisplatin, Fluoruracil; Adriamycin, Asparaginase, Azacitidin, Azathioprin, Bleomycin, Busulfan, Carboplatin, Carmustin, Chlorambucil, Cyclophosphamid, Cyclosporin, Cytarabin, Dacarbazin, Dactinomycin, Daunorubicin, Doxorubicin, Estramustin, Etoposid, Etretinat, Filgrastin, Floxuridin, Fludarabin, Fluoruracil, Florxymesteron, Flutamid, Goserelin, Hydroxyharnstoff, Ifosfamid, Leuprolid, Levamisol, Limustin, Nitrogen-Mustard, Melphalan, Mercaptopurin, Methotrexat, Mitomycin, Mitotan, Pentostatin, Pipobroman, Plicamycin, Procarbazin, Sargramostin, Streptozocin, Tamoxifen, Taxol, Teniposid, Thioguanin, Uracil-Mustard, Vinblastin, Vincristin und Vindesin; immunologische Arzneimittel, ausgewählt aus der Gruppe umfassend Impfstoffe und Immunstimulationsmittel; Insulin, Calcitonin, Nebenschilddrüsenhormon und Peptid und Vasopressin-Hypothalamus-Freisetzungsfaktor; Beta-Adrenolytika, ausgewählt aus der Gruppe umfassend Timolol, Levobunolol und Betaxolol; Cytokine, Interleukine und Wachstumsfaktoren, Epidermiswachstumsfaktor, Fibroblastenwachstumsfaktor, Plättchen-abgeleiteter Wachstumsfaktor, Transformationswachstumsfaktor-beta, ziliarer, neurotroper Wachstumsfaktor, Glia-abgeleiteter neurotropher Faktor, NGF, EPO, PLGF, Hirnnervenwachstumsfaktor (BNGF), vaskulärer endothelialer Wachstumsfaktor (VEGF) und monoklonale Antikörper, die gegen solche Wachstumsfaktoren gerichtet sind; Antientzündungsmittel, ausgewählt aus der Gruppe umfassend Hydrocortison, Dexamethason, Fluocinolon, Prednison, Prednisolon, Methylprednisolon, Fluorometholon, Betamethason und Triamcinolon; Dekongestiva, ausgewählt aus der Gruppe umfassend Phenylephrin, Naphazolin und Tetrahydrazolin; Miotika und Anticholinesterasen, ausgewählt aus der Gruppe umfassend Pilocarpin, Carbachol, Diisopropylfluorphosphat, Phospholin-lod, Demecariumbromid; Mydriatika, ausgewählt aus der Gruppe umfassend Atropinsulfat, Cyclopentolat, Homatropin, Scopolamin, Tropicamid, Eucatropin; Sympathomimetika, ausgewählt aus der Gruppe umfassend Epinephrin und Vasokonstriktoren und Vasodilatoren; Antiblutgerinnungsmittel, ausgewählt aus der Gruppe umfassend Heparin, Antifibrinogen, Fibrinolysin, Antiblutgerinnung-Aktivase, Antidiabetesmittel, ausgewählt aus der Gruppe umfassend Acetohexamid, Chlorpropamid, Glipizid, Glyburid, Tolazamid, Tolbutamid, Insulin und Aldosereduktaseinhibitoren, Hormone, Peptide, Nukleinsäuren, Saccharide, Lipide, Glykolipide, Glykoproteine und andere Makromoleküle umfassen Endokrinhormone, ausgewählt aus der Gruppe umfassend hypophysäres Hormon, Insulin, mit Insulin verwandten Wachstumsfaktor, Schilddrüsenhormon, Wachstumshormone; Hitzeschockproteine; Modifier der Immunantwort, ausgewählt aus der Gruppe umfassend Muramyldipeptid, Cyclosporine, Interferone, (einschließlich alpha-, beta- und gamma-Interferone), Interleukin-2, Cytokine, FK506 (ein Epoxy-pyrido-oxaacyclotricosin-tetron, ebenfalls bekannt als Tacrolimus), Tumomekrosefaktor, Pentostatin, Thymopentin, Transformationsfaktor beta.sub.2, Erythropoetin; Antineogeneseproteine (z.B. Anti-VEGF, Interferone); Antikörper (monoklonal oder polyklonal) oder Antikörperfragmente, Oligoaptamere, Aptamere und Genfragmente (Oligonukleotide, Plasmide, Ribozyme, kleine RNA-Interferenz (SiRNA), Nukleinsäurefragmente, Peptide); Immunmodulatoren, ausgewählt aus der Gruppe umfassend Endoxan, Thalidomid, Tamoxifen; Antithrombolytische und Vasodilatormittel, ausgewählt aus der Gruppe umfassend rtPA, Urokinase, Plasmin, Stickoxid-Donoren; Nukleinsäuren, optional exprimiert, um ein Protein zu produzieren, das eine Vielzahl von pharmakologischen, physiologischen oder immunologischen Aktivitäten haben kann.

## Revendications

1. Utilisation d'une composition comprenant :
- une émulsion cationique, qui est une émulsion de type huile/eau qui comprend des particules colloïdes ayant un noyau huileux entouré d'un film interfacial, le film comprenant des agents actifs de surface, des lipides ou les deux, lesdites émulsions étant **caractérisées en ce qu'**au moins une partie des agents actifs de surface ou lipides dans le film interfacial ont des groupes polaires positivement chargés et en outre **en ce que** les particules colloïdes ont un potentiel zéta positif, et
- éventuellement au moins un ingrédient pharmaceutique actif pour la fabrication d'un médicament dans une forme adaptée pour une administration intra- et péri-oculaire, afin de traiter des conditions intraoculaires choisies dans le groupe constitué d'une inflammation intraoculaire, une infection, une croissance cancéreuse, des tumeurs, une croissance néovasculaire provenant de la rétine et/ou des choroïdes, un oedème rétinien, un oedème maculaire, une rétinopathie diabétique, une rétinopathie de la prématurité, des maladies dégénératives de la rétine (dégénérescence maculaire, dystrophies rétiniennes), des maladies rétiniennes associées à la prolifération gliale, plus spécifiquement, les conditions oculaires comme le glaucome, la vitréo-rétinopathie proliférative, la rétinopathie diabétique, la dégénérescence maculaire liée à l'âge, l'uvéite, la rétinite par cytomégalovirus, les dystrophies rétiniennes virales de l'herpes simplex, par une administration intra-vitréale, péri-bulbaire, latéro-bulbaire, sous-conjonctivale, sous-tumorale et rétrobulbaire.

2. Utilisation selon la revendication 1, dans laquelle l'émulsion cationique comprend (exprimés en % en poids) : 0,5-20% d'excipient huileux, de préférence 0,5-10% ; 0,01-2% de tensioactifs cationiques ou lipides, de préférence 0,02-0,4% et éventuellement un tensioactif non ionique dans une gamme de 0,05-3%, de préférence dans une gamme de 0,1-2%.

3. Utilisation selon la revendication 2, dans laquelle l'émulsion comprend en outre 0,05-3% de phospholipides, de préférence 0,1-2%.

4. Utilisation selon l'une quelconque des revendications 1 à 3, dans laquelle le pH de l'émulsion est compris entre 4,0 et 8,5, de préférence entre 6,0 et 8,0.

5. Utilisation selon l'une quelconque des revendications 1 à 4, dans laquelle l'émulsion comprend en outre un additif choisi dans le groupe des régulateurs de pression osmotique, des antioxydants, des conservateurs, du dextrose, des excipients, des agents stabilisants, des agents mouillants, des améliorants de viscosité, des hydrogels.

6. Utilisation selon l'une quelconque des revendications 1 à 5, dans laquelle l'ingrédient actif est choisi dans le groupe des anesthésiques, analgésiques, agents de mobilité/transport cellulaire comme les colchicines, la vincristine, la cytochalasine B et les composés reliés ; des inhibiteurs d'anhydrase carbonique choisis dans le groupe comprenant de l'acétazolamide, du méthazolamide, du dichlorphénamide, du diamox et des neuroprotecteurs choisis dans le groupe comprenant la nimodipine et les composés reliés ; des antibiotiques choisis dans le groupe comprenant la tétracycline, la chlortétracycline, la bacitracine, la néomycine, la polymyxine, la gramicidine, la céphalexine, l'oxytétracycline, le chloramphénicol, la rifampicine, la ciprofloxacine, les aminosides, la gentamycine, l'érythromycine et la pénicilline, la quinolone, le ceftazidime, la vancomycine, l'imipénème ; des antifongiques choisis dans le groupe comprenant l'amphotéricine B, le fluconazole, le kétoconazole et le miconazole ; des antibactériens choisis dans le groupe comprenant des sulfonamides, la sulfadiazine, le sulfacétamide, le sulfaméthizole et le sulfisoxazole, le nitrofurazone et le propionate de sodium ; des antiviraux, choisis dans le groupe comprenant l'idoxuridine, la trifluorothymidiine, la trifluorouridine, l'acyclovir, le ganciclovir, le cidofovir, l'interféron, le DDI, l'AZT, le foscamet, la vidarabine, l'irbavirine, les inhibiteurs de protéase et les agents anti-cytomégalovirus ; des antihistaminiques choisis dans le groupe comprenant le cromoglycate de sodium, l'antazoline, la méthapyrilline, la chlorphéniramine, la cétirizine, la pyrilamine et la prophènepyridamine ; des glucocorticoïdes synthétiques et minéralo-corticoïdes et plus généralement des formes d'hormones dérivant du métabolisme du cholestérol (DHEA, progestérone, oestrogènes) ; des anti-inflammatoires non stéroïdiens choisis dans le groupe comprenant le salicylate, l'indométhacine, l'ibuprofène, le diclofénac, le flurbiprofène, le piroxicam et les inhibiteurs de COX2 ; des antinéoplasiques choisis dans le groupe comprenant la carmustine, la cisplatine, le fluorouracil ; l'adriamycine, l'asparaginase, l'azacitidine, l'azathioprine, la bléomycine, le busulfan, la carboplatine, la carmustine, le chlorambucil, le cyclophosphamide, la cyclosporine, la cytarabine, la dacarbazine, la dactinomycine, la daunorubicine, la doxorubicine, l'estramustine, l'étoposide, l'étrétinate, la filgrastine, la floxuridine, la fludarabine, le fluorouracil, la florxymestérone, le flutamide, la goséréline, l'hydroxy-urée, l'ifosphamide, la leuprolide, la lévamisole, la limustine, la moutarde azotée, le melphalan, la mercaptopurine, le méthotrexate, la mitomycine, le mitotane, la pentostatine, le pipobromane, la plicamycine, la procarbazine, la sargramostine, la streptozocine, le tamoxifène, le taxol, le téniposide, la thioguanine, la moutarde d'uracile, la vinblastine, la vincristine et la vindésine ; des médicaments immunologiques choisis dans le groupe comprenant des vaccins et stimulants immunitaires ; l'insuline, la calcitonine, l'hormone de parathyroïde et les peptides et facteurs libérant la vasopressine de l'hypothalamus; les bêtabloquants adrénergiques choisis dans le groupe comprenant le timolol, le lévobunolol et le betaxolol ; des cytokines, interleukines et facteurs de croissance, le facteur de croissance épidermique, le facteur de croissance fibroblastique, le facteur de croissance dérivé des plaquettes, le facteur de croissance transformateur bêta, le facteur de croissance neurotrophique ciliaire, le facteur neurotrophique dérivé de cellules gliales, le NGF, l'EPO, le PLFG, le facteur de croissance du nerf cérébral (BNGF), le facteur de croissance endothélial vasculaire (VEGF) et les anticorps monoclonaux dirigés contre ces facteurs de croissance ; des anti-inflammatoires choisis dans le groupe comprenant l'hydrocortisone, la dexaméthasone, la fluocinolone, la prednisone, la prednisolone, la méthylprednisolone, la fluorométholone, la bêtaméthasone et la triamcinolone ; des décongestionnants choisis dans le groupe constitué de phényléphrine, de naphazoline et de tétrahydrazoline ; des miotiques et anti-cholinestérases choisis dans le groupe comprenant la pilocarpine, le carbachol, le diisopropyl fluorophosphate, l'iode de phospholine et le bromure de démécarium ; des mydriatiques choisis dans le groupe comprenant le sulfate d'atropine, le cyclopentaolate, l'homatropine, la scopolamine, le tropicamide, l'eucatropine ; des sympathomimétiques choisis dans le groupe comprenant l'épinéphrine et les vasoconstricteurs et vasodilatateurs ; des agents anticoagulants choisis dans le groupe comprenant l'héparine, l'anti-fibrinogène, la fibrinolysine, l'activase anticoagulante, les agents antidiabétiques choisis dans le groupe comprenant l'acétohexamide, le chrlopropamide, le glipidzide, le glyburide, le tolazamide, le tolbutamide, l'insuline et des inhibiteurs de réductase d'aldose, des hormones, des peptides, des acides nucléiques, des saccharides, des lipides, des glycolipides, des glycoprotéines et d'autres macromolécules incluant des hormones endocrines choisis dans le groupe comprenant les hormones pituitaires, l'insuline, le facteur de croissance lié à l'insuline, la thyroïde, les hormones de croissance ; les protéines de choc thermique ; les modificateurs de réponse immunologique choisis dans le groupe comprenant du dipeptide de muramyle, des cyclosporines, des interférons (y compris des alpha-, bêta- et gamma-interférons), l'interleukine 2, les cytokines, le FK506 (une époxy-pyrido-oxaazycyclotricosine-tétrone, également connue sous le nom de Tacrolimus), le facteur de nécrose tumorale, la pentostatine, la thymopentine, le facteur de transformation bêta.sub.2, l'érythropoïétine, les protéines d'antinéogénèse (par exemple anti VEGF, interférons) ; des anticorps (monoclonaux ou polyclonaux) ou des fragments d'anticorps, des oligoaptamères, des aptamères et fragments géniques (oligonucléotides, plasmides, ribozymes, ARN de petite interférence (ARNSi), des fragments d'acide nucléique, des peptides) ; des immuno-modulateurs choisis dans le groupe comprenant endoxan, thalidomide, tamoxifène ; des agents antithrombolytiques et vasodilatateurs choisis dans le groupe comprenant le rtPA, l'urokinase, la plasmine, les donneurs d'oxyde nitrique ; des acides nucléiques éventuellement exprimés pour produire une protéine pouvant avoir une variété d'activités pharmacologiques, physiologiques ou immunologiques.
